# EUROPÄISCHE PATENTSCHRIFT

(11) **EP 0 849 266 B1**
(45) Veröffentlichungstag und Bekanntmachung des Hinweises auf die Patenterteilung: **14.02.2007**
(21) Anmeldenummer: 96120603.4
(22) Anmeldetag: 20.12.1996
(51) Int. Cl.: C07D 405/14, A61K 31/505

(54) **Neue polymorphe Form von Doxazosinmesylat (Form III)**
Novel polymorphic form of doxazosin mesylate (form III)
Nouvelle forme polymorphique de mésylate de doxazosin (forme III)

(43) Veröffentlichungstag der Anmeldung: 24.06.1998
(73) Patentinhaber: HEUMANN PHARMA GmbH, D-90478 Nürnberg (DE)
(72) Erfinder: Grafe, Ingomar Dr. Dipl.-Chem., 90482 Nürnberg (DE); Mörsdorf, Johann Peter Dr. Dipl.-Chem., 90579 Langenzenn (DE)
(74) Vertreter: Albrecht, Thomas

(56) Entgegenhaltungen:
- EP-A- 0 459 666
- WO-A-94/09783
- US-A- 4 188 390
- "The Merck Index" 1996 , MERCK & CO., INC. , WHITEHOUSE STATION, NJ XP002030968 * #3489: Doxazosin * * Seite 580 *

## Beschreibung

Die Erfindung betrifft eine neue, kristalline und wasserfreie Form von Doxazosin-Mesylat, ein Verfahren zu ihrer Herstellung und diese neue Form III enthaltende Arzneimittel.

1-(4-Amino-6,7-dimethoxy-2-chinazolyl)-4-[(2,3-dihydro-1,4-benzodioxin-2-yl)carbonyl]piperazinmethansulfonat mit dem INN-Namen Doxazosin-Mesylat ist ein Diaminochinazolinderivat aus der Klasse der α₁-Rezeptorenblocker. Es zeigt eine große strukturelle Ähnlichkeit zu den älteren Vertretern dieser Klasse, Prazosin-Hydrochlorid und Terazosin-Hydrochlorid. Während die beiden letztgenannten Wirkstoffe primär nur bei der Behandlung des Bluthochdrucks eingesetzt werden, eröffnet sich im Falle des Doxazosin-Mesylats eine weitere Indikation, nämlich die Behandlung der benignen Prostatahyperplasie.

Im Gegensatz zu Prazosin und Terazosin wird Doxazosin therapeutisch nicht als Hydrochlorid eingesetzt, sondern als Mesylat, d.h. als Salz der Methansulfonsäure.

Trotzdem bereits Doxazosin-Mesylat-enthaltende Arzneimittel im Handel sind, ist das Doxazosin-Mesylat bislang noch nicht beschrieben worden. Auch die US-PS 4 188 390, die erstmals Doxazosin offenbart, enthält keine Beschreibung von Doxazosin-Mesylat. In den Beispielen dieser Druckschrift wird lediglich das Doxazosin-Monohydrochlorid beschrieben.

Das Hydrochlorid ist jedoch wegen seiner extremen Schwerlöslichkeit in Wasser für pharmazeutische Zwecke ungeeignet.

Versuche zur Herstellung von Doxazosin-Mesylat auf den üblichen Wegen gestalten sich sehr schwierig und führen zu unbefriedigenden Ergebnissen. Einerseits ist Doxazosin-Base in den für Salzbildungen gängigen Lösungsmitteln schwer löslich. Lediglich in polaren, aprotischen, hochsiedenden Lösungsmitteln, wie beispielsweise Dimethylformamid, ist sie ausreichend löslich. Jedoch ist in diesen Lösungsmitteln die Löslichkeit des Doxazosin-Mesylats ähnlich der der Base, so daß die zu erhaltenden Ausbeuten an Mesylat absolut unbefriedigend sind. Außerdem ist Dimethylformamid aus pharmakologischer Sicht ein kritisches Restlösungsmittel in Arzneimittelwirkstoffen.

Andererseits kann Doxazosin-Base in schwachen Säuren, wie z.B. Essigsäure, gelöst werden und durch Zugabe von Methansulfonsäure das Mesylat gefällt werden. Jedoch erhält man bei dieser Arbeitsweise bei Raumtemperatur ein unfiltrierbares Gel. Arbeitet man bei höheren Temperaturen, beispielsweise 50°C, so klumpt dieses Gel zusammen oder ölt bei höheren Konzentrationen als zweite, nicht erstarrende Phase aus. Durch Zusatz organischer Lösungsmittel, beispielsweise Aceton, kann die Absaugfähigkeit des gefällten Doxazosin-Mesylats zwar verbessert werden, doch führt die Trocknung dieses Produkts zur Bildung von Klumpen aufgrund des hohen Feuchtegehalts. Letztendlich erhält man eine amorphe und hygroskopische Form des Doxazosin-Mesylats.

Der Erfindung liegt daher die Aufgabe zugrunde, eine kristalline und wasserfreie Form des Doxazosin-Mesylats bereitzustellen, die aufgrund ihrer physikalischen Eigenschaften, insbesondere ihrer Kristalleigenschaften und ihres Verhaltens gegen Wasser, sowohl in ihrer chemischen Herstellung als auch in der galenischen Formulierung gut zu handhaben ist.

Diese Aufgabe wird erfindungsgemäß durch eine neue, kristalline und wasserfreie Form des Doxazosin-Mesylats gelöst, die nachstehend als Form III bezeichnet wird.

Gegenstand der Erfindung ist daher die Form III von Doxazosin-Mesylat, die dadurch gekennzeichnet ist, daß sie ein Röntgen-Pulverdiagramm mit den folgenden Reflexlagen hoher und mittlerer Intensität:

| | |
|---|---|
| 8,49° | 21, 03° |
| 11,72° | 22,87° |
| 16,03° | 25,02° |
| 18,29° | |

aufweist und daß sie kristallin und wasserfrei ist.

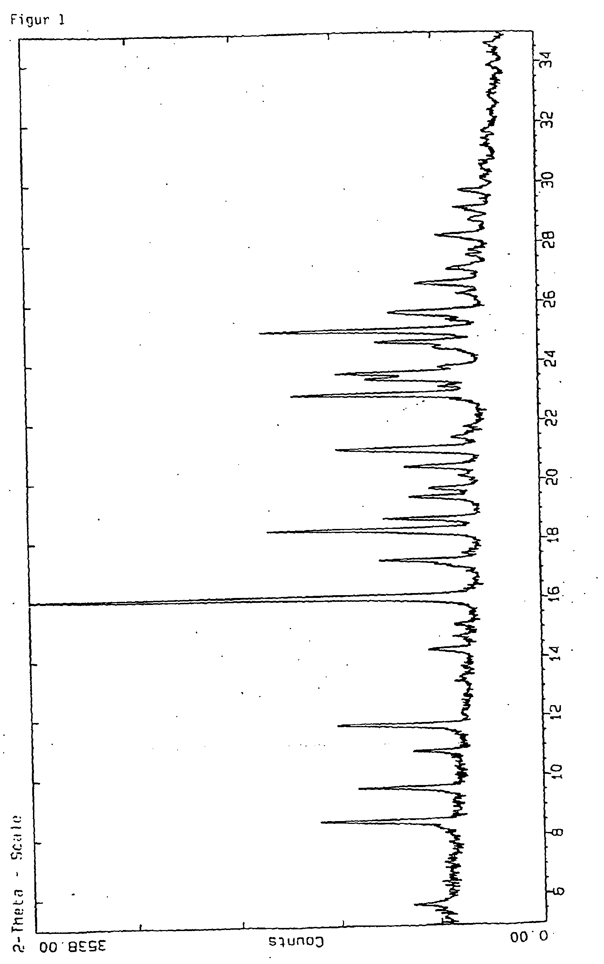

Die erfindungsgemäße Form III ist durch ein Röntgendiffraktogramm, gemessen unter Verwendung von Cu-K_{α1}-Strahlung und eines Ge-Monochromators mit einer Schrittweite von 0,017° im Beugungswinkelbereich von 2 θ = 5-35°, das in Figur 1 wiedergegeben ist und folgende Reflexlagen hoher und mittlerer Intensität aufweist, charakterisiert:

| | |
|---|---|
| 8, 49° | 21,03° |
| 11,72° | 22,87° |
| 16,03° | 25,02° |
| 18, 29° | |

Die erfindungsgemäße Form III von Doxazosin-Mesylat unterscheidet sich über das Röntgendiffraktogramm hinaus noch in einer Reihe von weiteren Eigenschaften von den anderen Formen des Doxazosin-Mesylats. Diese Eigenschaften können daher ebenfalls zur Unterscheidung von den anderen Formen herangezogen werden.

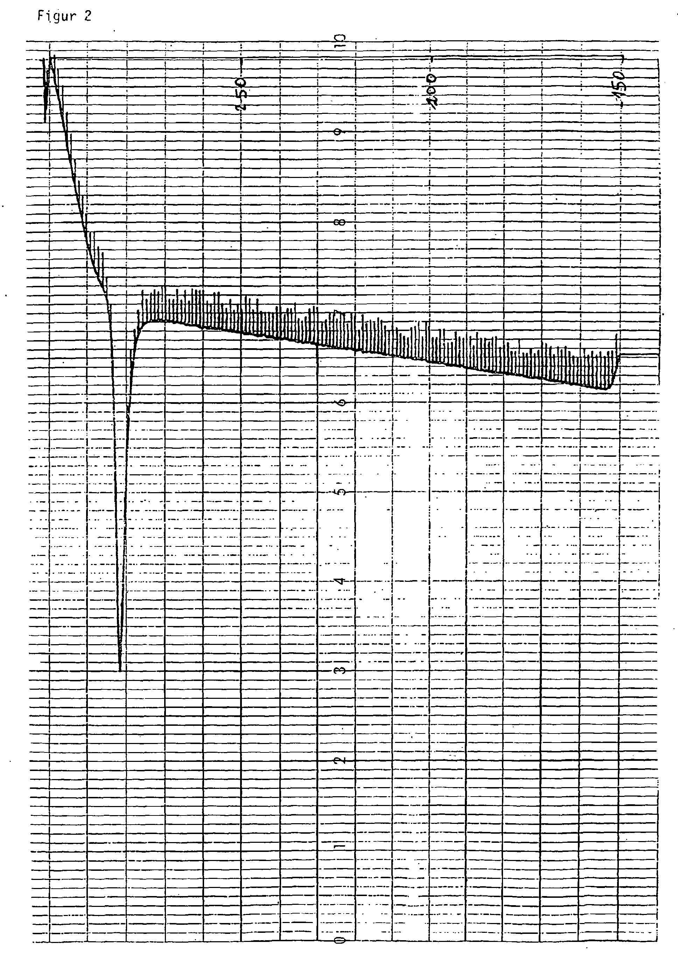

Eine weitere Charakterisierung der Form III des Doxazosin-Mesylats kann mit Hilfe der Differentialthermoanalyse (DTA) erfolgen. Gemäß dem in Figur 2 dargestellten DTA-Spektrum der Form III mit einem Meßbereich von 150 bis 300°C ist die Form III durch einen einzigen, endothermen Peak bei 281°C gekennzeichnet, der dem Schmelzpunkt der Form III entspricht.

Ein weiterer Gegenstand der Erfindung ist ein Verfahren zur Herstellung der oben beschriebenen, erfindungsgemäßen Form III von Doxazosin-Mesylat, das dadurch gekennzeichnet ist, daß man
(1) Doxazosinbase, gebenenfalls in einem organischen Lösungsmittel, mit Essigsäure zu Doxazosin-Acetat umsetzt,
(2) die in Stufe (1) erhaltene Lösung im heißen Zustand klärfiltriert und mit Methansulfonsäure versetzt,
(3) die in Stufe (2) erhaltene Lösung bei einer Temperatur von 30°C bis zur Rückflußtemperatur des verwendeten Lösungsmittels zur Kristallisation rührt und das so erhaltene Lösungsmitteladdukt durch Filtrieren gewinnt,
(4) das feuchte Lösungsmitteladdukt in einen niederen Alkohol einträgt und 10 Minuten bis 12 Stunden am Rückfluß kocht und daß man
(5) die in Stufe (4) erhaltene Lösung auf Raumtemperatur abkühlt und die ausgeschiedenen Kristalle durch Filtrieren gewinnt.

In der ersten Stufe des erfindungsgemäßen Verfahrens wird Doxazosin-Base mit Essigsäure zu Doxazosin-Acetat umgesetzt. Die Umsetzung kann in Abwesenheit eines Lösungsmittels oder vorzugsweise in einem organischen Lösungsmittel erfolgen. Beispiele für geeignete Lösungsmittel sind niedere Alkohole oder Ester, wobei ein Esterlösungsmittel, wie Ethylacetat, bevorzugt wird. Bei der Reaktion beträgt das Verhältnis von Doxazosin-Base zu Essigsäure 1:2 bis 1:5, vorzugsweise 1:2 bis 1:3. Die Reaktionstemperatur beträgt 40 bis 100°C, vorzugsweise 60 bis 90°C, insbesondere 80°C. Im Einzelfall hängt sie von der Zusammensetzung des Reaktionsgemisches ab.

Die in der ersten Stufe erhaltene Lösung wird noch im heißen Zustand klärfiltriert und mit Methansulfonsäure veretzt. Nach dem Waschen der Filterschicht mit einem heißen Gemisch aus Eisessig und einem organischen Lösungsmittel, vorzugsweise dem in Stufe 1 verwendeten Lösungsmittel, wobei das Volumenverhältnis vorzugsweise 1:1 beträgt, werden das Filtrat und die Waschflüssigkeit vereinigt und mit Methansulfonsäure versetzt. Die Methansulfonsäure wird, bezogen auf das Doxazosin-Acetat, äquimolar oder in geringem Überschuß von bis zu 10 Mol-%, vorzugsweise bis zu 7 Mol-% eingesetzt. Die Zugabe einer äquimolaren Menge von Methansulfonsäure wird bevorzugt. Die Methansulfonsäure wird vorzugsweise als 70%ige wäßrige Lösung eingesetzt.

In der nächsten Stufe des erfindungsgemäßen Verfahrens wird das noch lösungsmittel-feuchte Lösungsmitteladdukt in einen niederen Alkohol, wie Methanol oder Ethanol, vorzugsweise Methanol eingetragen und 10 Minuten bis 12 Stunden, vorzugsweise 6 bis 9 Stunden, am Rückfluß gekocht. Der niedere Alkohol wird dabei in einer Menge von 1:5 bis 1:20, bezogen auf das feuchte Lösungsmitteladdukt, eingesetzt.

In der letzten Stufe wird die auf die obige Weise erhaltene Lösung auf Raumtemperatur abgekühlt bis sich die angestrebte Verbindung vollständig in Form von Kristallen ausgeschieden hat. Die ausgeschiedenen Kristalle werden durch Filtration in üblicher Weise gewonnen.

Die erfindungsgemäße Form III des Doxazosin-Mesylats hat aufgrund ihrer kristallinen Eigenschaften überraschende Vorteile sowohl im Hinblick auf ihre Synthese und die Produktreinheit als auch für ihre galenische Verarbeitung zu festen Arzneiformen. Wie oben beschrieben, fallen die auf den üblichen Wegen erhaltenen Formen des Doxazosin-Mesylats in Form gelartiger, auch in Gegenwart von organischen Lösungsmitteln sehr voluminöser Niederschläge an, die große Mengen Mutterlaugen einschließen und daher Feuchtegehalte bzw. Trockenverluste von bis zu 50% aufweisen. Dadurch werden im getrockneten Produkt Verunreinigungen, insbesondere färbende Verunreinigungen, eingeschlossen bzw. adsorbiert. Desweiteren resultieren durch das gelartige voluminöse Produkt extrem lange Filtrations- bzw. Zentrifugationszeiten, die vom Prozeßstandpunkt her sehr nachteilig sind.

Dagegen wird die erfindungsgemäße Form III in Form eines gut kristallinen, farblosen und problemlos filtrier- bzw. zentrifugierbaren Feststoffs erhalten. Anhaftende Mutterlauge läßt sich durch Waschen des Filterkuchens mit einem geeigneten Lösungsmittel problemlos entfernen, so daß ein Produkt hoher Reinheit erhalten wird.

Amorphe Feststoffe, und erst recht hygroskopische Feststoffe, sind in der galenischen Verarbeitung sehr schlecht zu verarbeiten, da sie beispielsweise geringe Schüttdichten und mangelhafte Fließeigenschaften aufweisen. Außerdem sind zur Handhabung hygroskopischer Feststoffe spezielle Arbeitstechniken und Einrichtungen erforderlich, um reproduzierbare Ergebnisse, z.B. bezüglich des Wirkstoffgehalts oder der Stabilität in den produzierten Fertigarzneimitteln, zu erhalten.

Die erfindungsgemäße Form III des Doxazosin-Mesylats ist therapeutisch in der gleichen Weise anwendbar wie die Doxazosin-Base und ihre pharmazeutisch annehmbaren Säureadditionssalze, wie das im Handel befindliche Doxazosin-Mesylat mit nicht bekannten morphologischen Eigenschaften. Hauptindikationsgebiete sind die Behandlung des Bluthochdrucks sowie die Behandlung der benignen Prostatahyperplasie.

Ein weiterer Gegenstand der Erfindung ist daher ein Arzneimittel, das dadurch gekennzeichnet ist, daß es neben üblichen Hilfs- und Trägerstoffen die Form III von Doxazosin-Mesylat enthält.

So kann die erfindungsgemäße Form III des Doxazosin-Mesylats zu den üblichen Darreichungsformen mit Einschluß von peroralen und parenteralen Darreichungsformen formuliert werden. Bevorzugte Formulierungen sind Tabletten oder Kapseln. Sie können durch herkömmliche Mischverfahren und unter Anwendung herkömmlicher Hilfs- und Trägerstoffe, wie Bindemittel, Sprengmittel, Aromatisierungsmittel und dergleichen, hergestellt werden. Die Dosierung entspricht bekannten Formen von Doxazosin-Salzen.

Die nachstehenden Beispiele erläutern die Erfindung.

### Beispiel 1

### Herstellung der erfindungsgemäßen Form III von Doxazosin-Mesylat

In einem 1 1-Dreihalskolben werden 200 g Doxazosin-Base in 250 ml Eisessig und 200 ml Ethylacetat gelöst durch Erhitzen auf 80°C. Nach Zugabe von 23 ml Methansulfonsäure (70%) wird die heiße Lösung über eine Schicht von Supercel klärfiltriert, und die Filterschicht wird mit 30 ml heißem Eisessig/Ethylacetat (1:1) gewaschen. Zu den vereinigten Lösungen werden weitere 23 ml Methansulfonsäure (70%) gegeben und die Lösung.bei 50°C eine Stunde gerührt bis Kristallisation eintritt. Nach Kühlen auf 10°C und zweistündigem Rühren wird der ausgefallene Feststoff abgesaugt und mit 200 ml Ethylacetat gewaschen.

Das ethylacetat-feuchte Lösungsmitteladdukt wird anschließend in 1,0 1 Methanol eingetragen und 9 Stunden unter Rückfluß gekocht. Nach Abkühlen auf Raumtemperatur und Absaugen erhält man 240 g (99% d. Th.) eines farblosen Feststoffs, der das in Figur 1 gezeigte Röntgenbeugungsspektrum der Form III zeigt.

### Beispiel 2

### Herstellung der erfindungsgemäßen Form III von Doxazsoin-Mesylat

Das gemäß Beispiel 1 erhaltene, ethylacetat-feuchte Doxazosin-Mesylat wird in drei Volumenteilen Ethanol 3 Stunden unter Rückfluß gekocht. Nach Abkühlen auf Raumtemperatur und Absaugen erhält man wiederum einen farblosen Feststoff in praktisch quantitativer Ausbeute, der nach Röntgendiffraktogramm die Form III von Doxazosin-Mesylat ist und auch das in Figur 2 wiedergegebene DTA-Spektrum zeigt.

## Patentansprüche

1. Form III von Doxazosin-Mesylat, **dadurch gekennzeichnet, daß** sie ein Röntgen-Pulverdiagramm mit den folgenden Reflexlagen hoher und mittlerer Intensität:
| | |
|---|---|
| 8,49° | 21,03° |
| 11,72° | 22,87° |
| 16,03° | 25,02° |
| 18,29° | |
aufweist und daß sie kristallin und wasserfrei ist.

2. Verfahren zur Herstellung der Form III von Doxazosin-Mesylat nach Anspruch 1, **dadurch gekennzeichnet, daß** man
(1) Doxazosinbase, gegebenenfalls in einem organischen Lösungsmittel, mit Essigsäure zu Doxazosin-Acetat umsetzt,
(2) die in Stufe (1) erhaltene Lösung im heißen Zustand klärfiltriert und mit Methansulfonsäure versetzt,
(3) die in Stufe (2) erhaltene Lösung bei einer Temperatur von 30°C bis zur Rückflußtemperatur des verwendeten Lösungsmittels zur Kristallisation rührt und das so erhaltene Lösungsmitteladdukt durch Filtrieren gewinnt, .
(4) das feuchte Lösungsmitteladdukt in einen niederen Alkohol einträgt und 10 Minuten bis 12 Stunden am Rückfluß kocht und daß man
(5) die in Stufe (4) erhaltene Lösung auf Raumtemperatur abkühlt und die ausgeschiedenen Kristalle durch Filtrieren gewinnt.

3. Arzneimittel, **dadurch gekennzeichnet, daß** es neben üblichen Hilfs- und Trägerstoffen die Form III von Doxazosin-Mesylat nach Anspruch 1 enthält.

## Claims

1. Form III of doxazosin mesylate, **characterised in that** it shows an X-ray powder diagram having the following reflex positions of high and medium intensity:
| | |
|---|---|
| 8.49° | 21.03° |
| 11.72° | 22.87° |
| 16.03° | 25.02° |
| 18.29° | |
and **in that** it is crystalline and anhydrous.

2. A process for preparing the above Form III of doxazosin mesylate according to claim 1, which is **characterised in that**
(1) doxazosin base is converted with acetic acid into doxazosin acetate, optionally in an organic solvent,
(2) the solution obtained in stage (1) is clarified in the hot state and mixed with methanesulphonic acid,
(3) the solution obtained in stage (2) is stirred at a temperature from 30°C up to the reflux temperature of the solvent employed to achieve complete crystallisation and the solvent adduct so obtained is recovered by filtration,
(4) the moist solvent adduct is introduced into a lower alcohol and boiled at reflux for between 10 minutes and 12 hours and
(5) the solution obtained in stage (4) is cooled to room temperature and the precipitated crystals are recovered by filtration.

3. A medicament **characterised in that**, in addition to conventional auxiliary substances and carriers, it contains Form III of doxazosin mesylate according to claim 1.

## Revendications

1. Forme III de mésylate de doxazosine, **caractérisée en ce qu'**elle présente un diagramme aux rayons X pour surfaces pulvérulentes, avec les couches de réflexion suivantes de haute et de moyenne intensité :
| | |
|---|---|
| 8,49° | 21, 03° |
| 11,72° | 22,87° |
| 16,03° | 25,02° |
| 18,29° | |
et **en ce qu'**elle est cristalline et anhydre.

2. Procédé de production de la forme III de mésylate de doxazosine selon la revendication 1, **caractérisé en ce que**
(1) on transforme une base de doxazosine, le cas échéant dans un solvant organique avec de l'acide acétique en acétate de doxazosine,
(2) on clarifie la solution obtenue à l'étape (1) à l'état chaud et on la mélange avec de l'acide méthane sulfonique,
(3) on agite la solution obtenue à l'étape (2) à une température de 30°C jusqu'à la température de reflux du solvant utilisé pour la cristallisation, et on récupère ainsi le produit d'addition du solvant obtenu par filtration,
(4) on introduit le produit d'addition du solvant dans un alcool faible et on le porte à ébullition au reflux pendant de 10 minutes à 12 heures et **en ce que**
(5) on refroidit la solution obtenue à l'étape (4) à température ambiante et on récupère les cristaux déposés par filtration.

3. Produit pharmaceutique, **caractérisé en ce qu'**en plus des agents auxiliaires et des substances porteuses, il contient la forme III de mésylate de doxazosine selon la revendication 1.
